# EUROPEAN PATENT APPLICATION

(11) **EP 0 584 767 A1**
(43) Date of publication of application: **02.03.1994**
(21) Application number: 93113441.5
(22) Date of filing: 24.08.1993
(51) Int. Cl.: A61K 39/09

(54) **Rapid extraction and neutralization of streptococcal antigen**

(30) Priority: 26.08.1992 US 935755
(71) Applicant: Becton Dickinson and Company, Franklin Lakes, New Jersey 07417-1880 (US)
(72) Inventor: Smith, Mary Ann Nomsa, Baltimore, Maryland 21215 (US); Bloomster, Timothy, Shrewsbury, Pennsylvania 17361 (US)
(74) Representative: von Kreisler, Alek, Dipl.-Chem.

(57) **Abstract**

An improved, simple, and rapid method for extracting streptococcal antigens, preferably those of Group A Streptococci, from a sample is presented. The method comprises an improved micronitrous acid extraction procedure which eliminates the need for the separate addition of the nitrite and the neutralizing agent, by utilization of a unitized apparatus.

## Description

### BACKGROUND OF INVENTION

Streptococcal bacteria are important pathogens commonly associated with human diseases, particularly streptococci belonging to the Lancefield groups identified as Groups A, B, C, D, F, G and others. Of these, Group A streptococci are the most common infecting agent, but all groups are well-recognized as associated with important public health problems. If left untreated, serious health problems can result from infections by these bacteria.

Rapid diagnosis and treatment of Group A streptococcal pharangitis lessens the possibility of developing streptococcal sequelae, such as glomerulonephritis, rheumatic fever, rheumatic heart disease, etc. Other streptococci are associated with similarly serious complications. This has produced a continuing need for rapid uncomplicated, and sensitive procedures for the accurate diagnosis of streptococcal infections.

A wide array of immuno-assays have been developed to detect streptococcal antigens in the body fluids of patients. These assays involve contacting the antigens in a test sample with antibodies which are specific for reaction to the Group antigen. The antibody-antigen reaction is then monitored by methods which are well-known in the art.

For example, the antibody can be bound on the surface of minute particles. Reaction of the antigens and antibodies causes agglutination of the antibody coated particles forming agglutination complexes which are visible to the naked eye.

One well known agglutination technique of this type uses polystyrene latex particles. The latex particles are small, and thus "invisible" unless agglutinated. The antibody is attached to the surface of the latex particle by a method which does not interfere with the antigen-antibody interaction. When exposed to streptococcal antigen, the bound antibody reacts with the antigen, thus bonding the antigen to the antibody. A plurality of such reactions forms agglutination complex, which can then be visualized. Absence of the complex is indicative of absence of the antigen and, thus, a negative result.

Alternatively, the antibody can be conjugated with other labels to provide detection. These labels include enzymes, which react with a substrate to produce a detectable signal. Such detection systems are ordinarily called enzyme linked immunosorbent assays (ELISA) and are described, inter alia, in United States Patent Nos. 4,474,878 and 4,642,285 to Halbert et al.

Alternatively, a direct visual readout can be obtained by the use of visible particulate detectors such as those described in United States Patent No. 4,703,017 to Campbell et al.

In all of these test procedures, a critical requirement is the isolation of the antigenic groups from the streptococcal bacteria. Such isolation will serve to expose the antigens, thereby increasing the sensitivity of the assay, as well as the speed and ease of its performance.

A wide array of procedures for the isolation of streptococcal group specific carbohydrate antigens is well known in the art, including the use of high temperature or enzymes to disrupt the bacterial cell wall. However, the most widely used extraction procedure is that of the micronitrous acid extraction, described in Gerber, Journal of Clin. Micro., pp. 187-189, Jan. 1983, and in European Patent Application 0150567 to Meridian Diagnostics, Inc. published August 7, 1985, both incorporated herein by reference.

In this procedure, the major drawbacks of the previous extraction systems, namely the requirement of laborious time consuming procedures, are minimized. By utilizing a stable acid, such as acetic acid, and a nitrite salt (such as NaNO₂). Mixing of the acid with the nitrite salt generates nitrous acid (HNO₂) which is a relatively unstable oxidizing agent. Nitrous acid extracts the group specific carbohydrate antigens from the streptococcal cell wall. In practice, the sample is obtained on a swab (e.g. a throat swab) which is placed in a container. The required amounts of acid and aqueous nitrite are then added; after the prescribed time, an amount of base is added to bring the system to the described pH for the assay.

As such, it can be readily appreciated that a number of manipulations are needed to obtain the antigen in condition for the assay. As each manipulation requires the devotion of technician time and increases the chance of error and/or contamination, minimization of these manipulations is desirable.

### SUMMARY OF INVENTION

This invention presents an improved, simple, and rapid method for extracting streptococcal antigens, preferably those of Group A Streptococci, from a sample. Specifically, this invention presents an improved micronitrous acid extraction procedure which eliminates the need for the separate addition of the nitrite and the neutralizing agent.

Briefly, the nitrite is contained within the container (e.g. an extraction tube) on a pad or disc. Alternatively, the nitrite can be adsorbed directly on the surface of the tube, if the tube is constructed of a material to which it will adhere. After the sample and acid are added, the extraction is performed directly in the container. The container contents are then brought to the desired pH by squeezing or pouring the container contents through a tip or funnel containing a neutralizing agent or an ion exchange resin adsorbed on a filter pad or disc. The liquid, containing the antigen, can then be used in any subsequent assay.

### DETAILED DESCRIPTION OF INVENTION

This invention presents an improved version of the micronitrous acid extraction procedure described previously. The improvements permit the assay to be accomplished more rapidly, and obviate the need for multiple reagent additions. While the descriptions refer to the sample collection device as a "swab", this nomenclature is being used for convenience only, as swabs are the most common means for collection of Group A streptococcal samples. It is to be understood by the skilled artisan that any collection means not incompatible with the assay format selected could be used. Similarly, while the descriptions refer to human subjects, it is to be understood that the procedures could similarly be employed with samples collected from animals, so long as they are amenable to nitrous acid extraction.

The containers utilized are constructed of any material compatible with the reagents used herein, and are of a geometry and size sufficient to contain the sample collection device, the nitrite, and the additional acid, simultaneously. It is preferable that the container by constructed of a flexible substance, such as a plastic, if it is to be in conjunction with a cap containing the neutralizing agent, to facilitate the squeezing of the liquid through the cap. One such tube is marketed by Becton, Dickinson and Company under the trademark DispensTube®.

The container contains the nitrite salt, preferably sodium nitrite, deposited on a glass or other synthetic fiber disc. While it is preferred that this disc remain situated in the container by means of friction alone, the use of a compatible adhesive to hold it in place is permissible.

The neutralizing device can be in any convenient form, but is preferably in the form of a "funnel" or cap having a hole at one end. The use of a cap is especially preferred if the container is a flexible tube. In such a case, the cap is placed atop the tube where it is held by friction, screw threads, or other sealing means, and the liquid can be forced through the cap by squeezing the tube.

Regardless of shape, the neutralizing device contains a sufficient amount of neutralizing agent to bring the pH to the pH needed for the assay, preferably 6.5-9.5. The neutralizing agent is preferably a basic buffer, such as Tris, or an ion exchange resin such as BioRad 501 8x (mixed bed exchange resin). The agent can also be absorbed into glass fiber or synthetic fibers to facilitate use.

The acid added to the container can be any acid which is capable of reacting with the nitrite to form nitrous acid, and which will not deleteriously affect the antigen or pose toxicity problems to the user. Preferred acids include acetic acid, more preferably 0.5N acetic acid, and hydrochloric acid. The acid is added in a sufficient quantity to form the desired concentration of nitrous acid.

In the practice of a preferred embodiment of the invention, a specimen is collected on a throat swab by procedures well known in the art. The swab is then placed in a clean, dry tube containing the nitrite. Nitrous acid is formed by adding a sufficient quantity of glacial acetic acid to the tube. The nitrous acid serves to extract the antigen from the test sample on the swab. The cap containing the neutralizing agent is placed atop the tube and, after a sufficient period of time, the liquid is squeezed through the cap to bring the mixture to a pH of 6.5-9.5.

The effluent solution can then be applied to the test chamber or device to permit a subsequent assay to be run.

### EXAMPLES

The following examples illustrate certain preferred embodiments of the instant invention but are not intended to be illustrative of all embodiments.

### EXAMPLE 1

An 8mm diameter glass fiber disc was placed inside a DispensTube® where it was held near the bottom by friction. The disc was subsequently impregnated with 50µl of 4M aqueous sodium nitrite and oven dried at 45°C overnight.

A neutralizing cap (which fit the tube) was prepared by impregnating 3 layers of glass fiber with 2.6M aqueous Tris buffer and oven drying at 45°C.

A throat swab was placed in the tube and 600µl 0.5N acetic acid was added. After one (1) minute the swab was removed, the neutralizing cap was placed atop the tube, and the sample was squeezed through the cap onto a Directigen® 1-2-3 Group A Strep test device. The assay was run according to the procedure recited in the directions. The assay was run according to the procedure recited in the directions. A color change indicated a positive result.

For confirmation, swabs were plated and cultured on blood agar and strep selective agar, and any colonies were confirmed as Group A Strep by a latex agglutination immunoassay.

In a total of 180 replicates, the Directigen® 1-2-3 using this extraction procedures identified 31 positive results (culture showed 33) and 149 negatives (culture showed 147).

### EXAMPLE 2

In an alternative embodiment, the disc placed in the tube is hydrophylic foam impregnated with 50µl of 8M sodium nitrite. The neutralizing cap is similarly prepared, using 2M Tris (pH 9.5) on glass fiber and sandwiched between two layers of Porex. In all other ways, the extraction and neutralization will proceed as in Example 1.

### EXAMPLE 3

In another alternative embodiment the disc is the same as in example 2. The neutralizing cap is similarly prepared, except that felt is substituted for the Porex. In all other ways, the extraction and neutralization will proceed as in Examples 1 and 2.

It is apparent that many modifications and variations of this invention as hereinabove set forth may be made without departing from the spirit and scope hereof. The specific embodiments described are given by way of example only and the invention is limited only by the terms of the appended claims.

## Claims

1. A process for extracting streptococcal group specific carbohydrate antigens from a clinical specimen comprising:
(i) placing the specimen in a container containing a nitrite salt;
(ii) adding to said container an effective amount of a acid such that the nitrite salt reacts to form nitrous acid;
(iii) permitting said nitrous acid to react with said specimen for a sufficient amount of time to extract said streptococcal specific carbohydrate antigens; and
(iv) passing said nitrous acid containing the streptococcal group specific carbohydrate antigens through a neutralizing agent to adjust the pH to a desired level.

2. The method of Claim 1 wherein the nitrite salt is sodium nitrite.

3. The method of Claim 1 wherein the acid is hydrochloric acid or acetic acid.

4. The method of Claim 1 wherein the neutralizing agent is absorbed into glass fibers or synthetic fibers.

5. The method of Claim 1 wherein the container is a extraction tube constructed of flexible plastic.

6. The method of Claim 5 further characterized in that the neutralizing agent is contained within a cap having a hole at one end, which can be mounted atop the tube.

7. A device for extracting streptococcal group specific carbohydrate from a clinical specimen comprising:
(i) a container adapted to contain the specimen which contains a nitrite salt;
(ii) means for introducing an effective amount of an acid such that the nitrite salt reacts to form nitrous acid;
(iii) means for exposing said specimen to said nitrous acid; and
(iv) means for passing said nitrous acid containing said streptococcal group specific antigen through a neutralizing agent to adjust the pH to a desired level.

8. The device of Claim 7 wherein the nitrite salt is sodium nitrite.

9. The device of Claim 7 wherein the container is a tube constructed of flexible plastic.

10. The device of Claim 9 further characterized in that the neutralizing agent is contained within a cap having a hole at one end, which can be mounted atop the tube.
